# EUROPEAN PATENT APPLICATION

(11) **EP 3 593 801 A1**
(43) Date of publication of application: **15.01.2020**
(21) Application number: 18763356.5
(22) Date of filing: 06.03.2018
(51) Int. Cl.: A61K 31/445, A61K 9/107, A61K 47/10, A61K 47/14, A61K 47/18, A61P 23/02

(54) **ACIDIC EMULSION COMPOSITION CONTAINING LOCAL ANESTHETIC AGENT**

(30) Priority: 06.03.2017 JP 2017042253
(71) Applicant: Maruishi Pharmaceutical Co., Ltd., Osaka 538-0042 (JP)
(72) Inventor: OKAMOTO, Kazuki, Osaka-shi Osaka 538-0042 (JP); MASUI, Kuniharu, Osaka-shi Osaka 538-0042 (JP); NOMURA, Mai, Osaka-shi Osaka 538-0042 (JP); CHIDA, Yuichiro, Osaka-shi Osaka 538-0042 (JP)
(74) Representative: Teipel, Stephan
(86) International application number: PCT/JP2018/008572
(87) International publication number: WO 2018/164121

(57) **Abstract**

An object of the present invention is to provide a local anesthetic-containing composition characterized by (1) exerting immediate and long-lasting medicinal effect without sustained release after administration, and/or (2) having storage stability.

The present invention provides an acidic emulsion compos ition comprising a local anesthetic and a glyceride in which a fatty acid(s) having 6 to 12 carbon atoms is/are bound to glycerin via an ester bond(s).

## Description

### TECHNICAL FIELD

The present invention relates to a local anesthetic-containing acidic emulsion composition characterized by (1) exerting immediate and long-lasting medicinal effect without sustained release after administration, and/or (2) having storage stability, and use of the composition for pain relief. As used herein, the term "sustained release" means gradual release of a medicinal substance, resulting in, for example, slow onset and long duration of action of and/or long-term maintenance of the blood level of the medicinal substance.

### BACKGROUND ART

Various local anesthetic compounds have been developed, and most of them are clinically applied in the form of a salt in an aqueous solution preparation as appropriate for their respective properties. Meanwhile, to control the duration of action of local anesthetics, various sustained release techniques, exemplified by encapsulation of a high-concentration medicinal substance in PLGA (lactic acid/glycolic acid copolymer) or liposomes, and dissolution of a medicinal substance in oil bases, have been proposed (see Patent Literature 1 to 4 and Non Patent Literature 1). In particular, the sustained release technique using oil bases is a technique which generally utilizes the lipophilicity of local anesthetics to enhance their dissolution in the oily phase. To this end, this technique adopts an alkaline pH range, which is different from that of aqueous solution preparations containing local anesthetics in the form of a salt.

Emulsions are used as a carrier for lipophilic medicinal substances. For example, propofol injection, which is an intravenous general anesthetic, flurbiprofen axetil injection, which is a nonsteroidal analgesic, etc. are marketed in the form an emulsion.

Pain management using local anesthetics including postoperative analgesia is recognized as highly important for Enhanced Recovery After Surgery (ERAS), as with early initiation of rehabilitation etc. However, the duration of action of many local anesthetics is not as controllable as desired in clinical practice, and there is a strong need for compositions which are more excellent in controlling the duration of action without sacrificing the fast onset of action. This need is increasingly growing with the progress of peripheral nerve block techniques using high-resolution ultrasonic guidance.

### CITATION LIST

### Patent Literature

Patent Literature 1: JP-W 2001-516714
Patent Literature 2: Japanese Patent No. 3701693
Patent Literature 3: European Patent No. 0770387
Patent Literature 4: WO 2015-132985

### Non Patent Literature

Non Patent Literature 1: Henrik Dyhre et al., "Local Anesthetics in Lipid-Depot Formulations-Neurotoxicity in Relation to Duration of Effect in a Rat Model", Regional Anesthesia and Pain Medicine, Vol 31, No. 5 (September-October), 2006, 401-408

### SUMMARY OF INVENTION

### TECHNICAL PROBLEM

An object of the present invention is to provide a local anesthetic-containing composition characterized by (1) exerting immediate and long-lasting medicinal effect without sustained release after administration, and/or (2) having storage stability.

### SOLUTION TO PROBLEM

That is, the present invention relates to the following.
[1] An acidic emulsion composition comprising a local anesthetic and a fatty acid ester containing an ester-bonded fatty acid having 6 to 12 carbon atoms.
[2] The acidic emulsion composition according to the above [1], wherein the fatty acid ester is a glyceride.
[3] The acidic emulsion composition according to the above [1] or [2], wherein the local anesthetic is an amide-type local anesthetic.
[4] The acidic emulsion composition according to any one of the above [1] to [3], wherein the local anesthetic is one or more selected from the group consisting of levobupivacaine, bupivacaine, ropivacaine and salts thereof.
[5] The acidic emulsion composition according to any one of the above [2] to [4], wherein the glyceride is one or more fatty acid glycerides comprising one or more fatty acids having 6 to 12 carbon atoms as a constituent fatty acid, and is selected from the group consisting of a fatty acid monoglyceride, a fatty acid diglyceride and a fatty acid triglyceride.
[6] The acidic emulsion composition according to any one of the above [1] to [5], wherein the emulsion composition has a pH of 3 or higher but lower than 7.0.
[7] The acidic emulsion composition according to any one of the above [1] to [5], wherein the emulsion composition has a pH of 3 to 6.5.
[8] The acidic emulsion composition according to any one of the above [1] to [5], wherein the emulsion composition has a pH of 3 to 6.0.
[9] The acidic emulsion composition according to any one of the above [1] to [8], further comprising EDTA.
[10] The acidic emulsion composition according to any one of the above [1] to [9], further comprising a long-chain fatty acid glyceride.
[11] The acidic emulsion composition according to any one of the above [1] to [10], wherein the emulsion is an O/W type emulsion.
[12] The acidic emulsion composition according to the above [11], wherein the proportion of the local anesthetic in an aqueous phase is 15% or more of the total amount of the local anesthetic in the composition.
[13] The acidic emulsion composition according to any one of the above [1], [3], [4], and [6] to [12], wherein the fatty acid ester is a propylene glycol fatty acid ester.
[14] The acidic emulsion composition according to any one of the above [1] to [13] for use in pain control in a mammal.
[15] The composition for use according to the above [14], wherein the pain control is achieved by systemic or local administration via a transdermal route, a subcutaneous route, an intracutaneous route, an intramuscular route, a perineural route, an intrapulpal route, an intraspinal route, an epidural route, an intravenous route, or a transmucosal route such as eye mucosa etc.
[16] The composition for use according to the above [14], wherein the pain control is achieved by conduction anesthesia, infiltration anesthesia or topical anesthesia.
[17] A method for controlling pain in a mammal, comprising the step of administering the acidic emulsion composition according to any one of the above [1] to [13] to a mammal.
[18] The method according to the above [17], wherein the pain control is achieved by systemic or local administration via a transdermal route, a subcutaneous route, an intracutaneous route, an intramuscular route, a perineural route, an intrapulpal route, an intraspinal route, an epidural route, an intravenous route, or a transmucosal route such as eye mucosa etc.
[19] The method according to the above [17], wherein the pain control is achieved by conduction anesthesia, infiltration anesthesia or topical anesthesia.
[20] Use of the acidic emulsion composition according to any one of the above [1] to [13] for production of a medicament for pain control.
[21] The use according to the above [20], wherein the pain control is achieved by systemic or local administration via a transdermal route, a subcutaneous route, an intracutaneous route, an intramuscular route, a perineural route, an intrapulpal route, an intraspinal route, an epidural route, an intravenous route, or a transmucosal route such as eye mucosa etc.
[22] The use according to the above [20], wherein the pain control is achieved by conduction anesthesia, infiltration anesthesia or topical anesthesia.
[23] Use of the acidic emulsion composition according to any one of the above [1] to [13] for pain control.
[24] The use according to the above [23], wherein the pain control is achieved by systemic or local administration via a transdermal route, a subcutaneous route, an intracutaneous route, an intramuscular route, a perineural route, an intrapulpal route, an intraspinal route, an epidural route, an intravenous route, or a transmucosal route such as eye mucosa etc.
[25] The use according to the above [23], wherein the pain control is achieved by conduction anesthesia, infiltration anesthesia or topical anesthesia.
[26] A medicament for pain control comprising the acidic emulsion composition according to any one of the above [1] to [13] .
[27] The medicament according to the above [26], wherein the pain control is achieved by systemic or local administration via a transdermal route, a subcutaneous route, an intracutaneous route, an intramuscular route, a perineural route, an intrapulpal route, an intraspinal route, an epidural route, an intravenous route, or a transmucosal route such as eye mucosa etc.
[28] The medicament according to the above [26], wherein the pain control is achieved by conduction anesthesia, infiltration anesthesia or topical anesthesia.

### ADVANTAGEOUS EFFECTS OF INVENTION

The present invention provides a local anesthetic-containing composition characterized by (1) exerting immediate and long-lasting medicinal effect without sustained release after administration, (2) having storage stability, and/or (3) having a high level of safety.

### BRIEF DESCRIPTION OF DRAWINGS

Fig. 1 shows the results of the assessment of the duration of local anesthetic effect over 420 minutes after administration of the samples of Example 1 and Comparative Example 1 to guinea pigs.
Fig. 2 shows the results of the assessment of the duration of local anesthetic effect over 540 minutes after administration of the samples of Examples 2 and 3 and Comparative Example 2 to guinea pigs.
Fig. 3 shows the time-course profile of drug release from the samples of Example 4 and Comparative Example 2.
Fig. 4 shows the change in blood level over 360 minutes after administration of the samples of Example 4 and Comparative Examples 2 and 3 to rats.
Fig. 5 shows the results of the assessment of the duration of local anesthetic effect over 360 minutes after administration of the samples of Example 4 and Comparative Examples 2 and 3 to rats.
Fig. 6 shows the results of the assessment of the duration of local anesthetic effect after administration of the samples of Examples 6 to 8 and Comparative Example 2 to rats.

### DESCRIPTION OF EMBODIMENTS

### Active ingredient

The emulsion composition of the present invention comprises a local anesthetic as an active ingredient.

Examples of the local anesthetic include amide-type local anesthetics and ester-type local anesthetics. Preferred are amide-type local anesthetics, for example, local anesthetic compounds containing a nitrogen-containing heterocyclic compound represented by the following general formula (I): 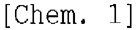 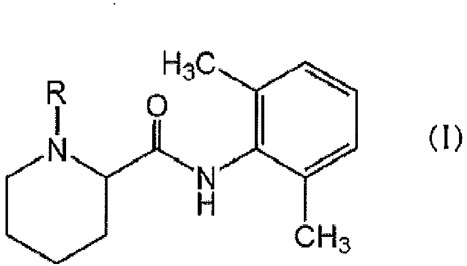 (wherein R represents a straight-chain or branched alkyl group having 1 to 4 carbon atoms), or one or more pharmaceutically acceptable salts thereof.

In the compound represented by the general formula (I), the carbon atom at which the carbonyl group is bound to the piperidine ring is a chiral carbon atom. The absolute configuration of the compound may be R or S.

In the case where the compound represented by general formula (I) in the present invention has one chiral carbon atom, the compound may be an optically pure compound with the R or S absolute configuration, a mixture of such optical isomers at any ratio, or a racemic mixture of such optical isomers.

In the case where the compound represented by the general formula (I) has two chiral carbon atoms, the compound may be an optically pure diastereomer, a racemic mixture of such diastereomers, or a mixture of such diastereomers at any ratio.

In the general formula (I), R is, for example, a methyl group, an ethyl group, a n-propyl group, an isopropyl group, a n-butyl group, an isobutyl group, a sec-butyl group, a tert-butyl group, or the like. R is preferably a straight-chain alkyl group having 1 to 4 carbon atoms. R is more preferably a straight-chain alkyl group having 2 to 4 carbon atoms, still more preferably a straight-chain alkyl group having 3 or 4 carbon atoms, and yet still more preferably a straight-chain alkyl group having 4 carbon atoms.

Examples of the local anesthetic include local anesthetics whose partition coefficient (n-octanol/pH 7.4 buffer solution) is 50 or more, and preferably 100 or more. The partition coefficient can be calculated according to a known method or with reference to known literature (e.g., Tucker. GT et al., Neural Blockade in Clinical Anesthesia and Management of Pain, Third Edition, 2008 p. 55, etc.).

Examples of the local anesthetic also include levobupivacaine (generic name) (chemical name: (2S)-1-butyl-N-(2,6-dimethylphenyl)piperidine-2-carboxamide ), bupivacaine (generic name) (chemical name: (2RS)-1-butyl-N-(2,6-dimethylphenyl)piperidine-2-carboxamid e), ropivacaine (generic name) (chemical name: (S)-N-(2,6-dimethylphenyl)-1-propylpiperidine-2-carboxamide ), and salts thereof, and mixtures thereof.

The salt is not particularly limited as long as it is a pharmaceutically acceptable salt. Examples include a pharmaceutically acceptable acid addition salt, a pharmaceutically acceptable metal salt, a pharmaceutically acceptable ammonium salt, a pharmaceutically acceptable organic amine addition salt, and a pharmaceutically acceptable amino acid addition salt. Examples of the acid addition salt include inorganic acid salts such as hydrochlorides, nitrates, sulfates, and phosphates; and organic acid salts such as oxalates, acetates, trifluoroacetates, maleates, fumarates, tartrates, citrates, lactates, malates, succinates, gluconates, ascorbates, and p-toluenesulfonates. In particular, preferred are acid addition salts, organic acid salts, etc., and more preferred are hydrochlorides.

The local anesthetic of the present invention is preferably a water-soluble salt.

As the local anesthetic exemplified above, a commercially available active substance may be used. Also, a pharmaceutical preparation containing the active substance can be used directly or after processing, for example, dilution, extraction, etc.

The amount of the local anesthetic is not particularly limited and is usually 0.01 to 5% by mass, preferably 0.01 to 3% by mass, particularly preferably 0.05 to 2% by mass relative to the whole composition.

The emulsion composition of the present invention may comprise an additional active ingredient unless this compromises the effects of the present invention. The additional active ingredient may be any appropriate substance that is not contraindicated for use with the local anesthetic.

### Fatty acid ester

The emulsion composition of the present invention comprises a fatty acid ester containing an ester-bonded fatty acid(s) having 6 to 12 carbon atoms. Preferable examples of the fatty acid ester include a glyceride in which a fatty acid(s) having 6 to 12 carbon atoms is/are bound to glycerin via an ester bond (s) and a propylene glycol fatty acid ester in which a fatty acid(s) having 6 to 12 carbon atoms is/are bound to propylene glycol via an ester bond(s).

The fatty acid having 6 to 12 carbon atoms may be a fatty acid having 6, 7, 8, 9, 10, 11 or 12 carbon atoms (e.g., caproic acid, enanthic acid, caprylic acid, pelargonic acid, capric acid or lauric acid), or a combination thereof.

### (1) Glyceride

The glyceride may comprise one or more fatty acids having 6 to 12 carbon atoms as a constituent fatty acid(s) at any ratio and may be a single kind or a combination of two or more kinds selected from a fatty acid monoglyceride, a fatty acid diglyceride and a fatty acid triglyceride. Preferred is a medium-chain fatty acid triglyceride (hereinafter, may be called "MCT" and preferably has a constituent fatty acid having 8 or 10 carbon atoms). The amount of the glyceride is not particularly limited unless it hinders the effects of the present invention. The amount of the glyceride is usually 0.01 to 20% by mass, preferably 0.5 to 15% by mass, and particularly preferably 1 to 10% by mass relative to the whole composition.

The glyceride may be produced by a known method. Alternatively, commercial products of medium-chain fatty acid triglycerides, such as COCONARD (trademark) MT (manufactured by Kao Corporation; a triglyceride having caprylic acid and capric acid as main constituent fatty acids); COCONARD RK (manufactured by Kao Corporation; a triglyceride having caprylic acid as a main constituent fatty acid); Captex 1000 (manufactured by Abitec; a triglyceride having capric acid as a main constituent fatty acid) ; Sunfat GDC-S (manufactured by Taiyo Kagaku Co., Ltd.; a diglyceride having caprylic acid as a main constituent fatty acid); and PANACET (Japanese registered trademark, manufactured by NOF CORPORATION), may also be used.

The constituent fatty acid may be a saturated fatty acid or an unsaturated fatty acid, and is preferably a saturated fatty acid.

### (2) Propylene glycol fatty acid ester

The propylene glycol fatty acid ester may comprise one or more fatty acids having 6 to 12 carbon atoms as a constituent fatty acid(s) at any ratio and may be a single kind or a combination of two or more kinds selected from a propylene glycol fatty acid monoester and a propylene glycol fatty acid diester. The propylene glycol fatty acid ester preferably has a constituent fatty acid having 8 or 10 carbon atoms . The amount of the propylene glycol fatty acid ester is not particularly limited unless it hinders the effects of the present invention. The amount of the propylene glycol fatty acid ester is usually 0.01 to 20% by mass, preferably 0.5 to 15% by mass, and particularly preferably 1 to 10% by mass relative to the whole composition.

The propylene glycol fatty acid ester may be produced by a known method. Alternatively, commercial products, such as Captex 100 (manufactured by Abitec; a propylene glycol diester having capric acid as a main constituent fatty acid), may also be used.

As with the glyceride, the propylene glycol fatty acid ester also has an ester bond(s) in the molecule.

The constituent fatty acid may be a saturated fatty acid or an unsaturated fatty acid, and is preferably a saturated fatty acid.

### pH

The pH of the emulsion composition of the present invention is adjusted to fall within an acidic range of less than 7.0, preferably 3 or higher but lower than 7.0, more preferably 3 to 6.5, and still more preferably 3 to 6.0.

When the pH of the emulsion composition of the present invention is adjusted to fall within the above range, the local anesthetic is less likely to be present in the oil phase and/or more likely to be present in the aqueous phase. In addition, when the pH of the emulsion composition of the present invention is adjusted to fall within the above range, the effects of the present invention, namely, (1) exerting immediate and long-lasting medicinal effect without sustained release after administration, (2) having storage stability, and/or (3) having a high level of safety, can be achieved and/or further improved.

### Emulsifier

The emulsifier used in the emulsion composition of the present invention is not particularly limited, and examples include deoxycholic acid, polyoxyethylene castor oil, polyoxyethylene hydrogenated castor oil, polyoxyethylene polyoxypropylene glycol, polyoxyethylene sorbitan monooleate (polysorbate 80), polyoxyethylene sorbitan monolaurate, polyoxyethylene hydroxystearate, povidone and lecithin (yolk lecithin, soybean lecithin). Preferred is lecithin, and more preferred is yolk lecithin. The amount of the emulsifier is preferably 0.1 to 3.0% by mass, more preferably 0.5 to 2.0% by mass, and particularly preferably 1.0 to 1.5% by mass relative to the whole emulsion composition.

### EDTA

The emulsion composition of the present invention may or may not further comprise a stabilizing agent. The stabilizing agent is not particularly limited and is, for example, EDTA, a fatty acid such as sodium oleate, cholesterol, casein, sodium caseinate, or the like. EDTA is effective for the improvement of emulsification stability and visual appearance of the emulsion composition and therefore is a preferable stabilizing agent. Here, the improvement of visual appearance means, for example, the prevention of time-dependent color change (e.g., reddish change) of the emulsion composition. EDTA may be EDTA-2Na, EDTA-4Na, EDTA-Ca-2Na, or any of hydrates thereof. The amount of EDTA is preferably 0.001 to 0.1% by mass, more preferably 0.001 to 0.05% by mass, and particularly preferably 0.01 to 0.05% by mass in terms of free EDTA relative to the whole emulsion composition. EDTA may be produced by a known method. Alternatively, commercial products, such as CLEWAT (Japanese registered trademark) N (EDTA-2Na-2H₂O, Nagase ChemteX Corporation), may also be used.

### Fat

The emulsion composition of the present invention may or may not further comprise a long-chain fatty acid glyceride. The number of carbon atoms of the long-chain fatty acid glyceride may be, for example, about 14 to 20. The amount of the long-chain fatty acid glyceride is not particularly limited unless it hinders the effects of the present invention. The amount of the long-chain fatty acid glyceride is usually 2 to 20% by mass, preferably 3 to 15% by mass, and particularly preferably 3 to 10% by mass relative to the whole composition.

The long-chain fatty acid glyceride may be produced by a known method. Alternatively, commercial products, such as purified soybean oil, may also be used.

### Solvent

The emulsion composition of the present invention may further comprise a solvent. The solvent is not particularly limited, and examples include purified water, distilled water, water for injection, glycerin, ethanol, propylene glycol, polyethylene glycol, macrogol and edible oils (sesame oil, corn oil, olive oil, etc.). In particular, preferred are purified water, distilled water, water for injection, etc.

The amount of the solvent is not particularly limited unless it hinders the effects of the present invention. The amount of the solvent is usually 80 to 99.9% by mass, preferably 80 to 98% by mass, and particularly preferably 80 to 95% by mass relative to the whole composition.

### Isotonic agent

The emulsion composition of the present invention may further comprise an isotonic agent. The isotonic agent is not particularly limited, and examples include glucose, D-sorbitol, sodium chloride, D-mannitol and glycerin. Preferred is glycerin. The amount of glycerin that renders the whole emulsion composition isotonic can be determined by calculation.

### Additive

The emulsion composition of the present invention preferably comprises an additive if desired. Examples of the additive include a solubilizer and an antioxidant.

### Additional medicinal ingredient

The emulsion composition of the present invention may comprise an additional medicinal ingredient. The additional medicinal ingredient is not particularly limited unless it adversely affects the emulsion composition, for example, reduces anesthetic effect or causes the instability of pharmaceuticals. The additional medicinal ingredient that may be comprised is, for example, dexamethasone, an ester thereof or a salt thereof (preferably, dexamethasone sodium phosphate), adrenaline, dexmedetomidine or a salt thereof (preferably, dexmedetomidine hydrochloride), or the like. By blending such an additional medicinal ingredient, an emulsion composition having a prolonged duration of action can be provided.

The solubilizer is not particularly limited, and examples include propylene glycol, D-mannitol, benzyl benzoate, ethanol, triethanolamine, sodium carbonate and sodium citrate.

Examples of the antioxidant include t-butylhydroquinone, butylated hydroxyanisole, butylated hydroxytoluene, L-cysteine hydrochloride, sodium hydrogen sulfite, α-tocopherol, polyphenol, ascorbic acid and derivatives thereof.

In the emulsion composition of the present invention, one of the above additives or a combination of two or more of them may be used as appropriate for the desired dosage form. The additives used may be commercial products. In the case where the emulsion composition comprises one or more additives, the amount of the additives is preferably 0.001 to 5% by mass, more preferably 0.001 to 3% by mass, and particularly preferably 0.01 to 3% by mass relative to the whole emulsion composition. When the amount of the additives is within the above range, these ingredients can usually fully exert their respective effects without sacrificing the effects of the present invention.

### Preferable combination

In a preferable embodiment, the emulsion composition of the present invention comprises a local anesthetic, an emulsifier, a medium-chain fatty acid triglyceride or a propylene glycol fatty acid ester, and water (e.g., distilled water, purified water, water for injection, or the like), and further comprises one or more ingredients selected from the group consisting of a long-chain fatty acid triglyceride, glycerin and EDTA.

### Production method

The emulsion composition of the present invention can be produced, for example, by adding a local anesthetic, a medium-chain fatty acid triglyceride or a propylene glycol fatty acid ester, and the other ingredients to water for injection in no particular order, stirring the mixture with heating, and homogenizing the whole. The temperature during the stirring is, for example, 50 to 80°C. That is, the emulsion composition of the present invention can be produced by a known method or its modified method.

### Properties

The emulsion composition of the present invention may be a W/O type emulsion or an O/W type emulsion, and is preferably an O/W type emulsion. The proportion of the local anesthetic in the aqueous phase of the emulsion composition to the whole of the local anesthetic in the emulsion composition is usually 10% or more, preferably 15% or more, and more preferably 30% or more. When the proportion of the local anesthetic in the aqueous phase of the emulsion composition to the whole of the local anesthetic in the emulsion composition is within the above range, the effects of the present invention, namely, (1) exerting immediate and long-lasting medicinal effect without sustained release after administration, (2) having storage stability, and/or (3) having a high level of safety, can be further improved.

For example, in a preferable embodiment of the emulsion composition of the present invention, during storage at 25°C and at a relative humidity of 60%, the amount of the local anesthetic is maintained at 90% or more of the initial level for 6 months or longer, and during the production and storage of the emulsion composition, no precipitates are formed. The composition of the present invention is excellent in storage stability.

### Method for use

The emulsion composition of the present invention can be used for pain control in, for example, humans and other mammals (e.g., rats, mice, guinea pigs, rabbits, sheep, pigs, cows, cats, dogs, monkeys, etc.).

Since the emulsion composition of the present invention is safe and low toxic, an adequate amount of the emulsion composition can be administered to, for example, humans and other mammals.

The dose varies with the type of the local anesthetic, the administration subject, the target organ, the symptom, the administration method, etc. For example, in the case of injectable preparations, it is usually convenient to administer the medicinal ingredient in a daily dose of, for example, about 0.01 to 1000 mg, preferably about 0.01 to 500 mg to a human weighing about 60 kg by a known administration method. The total daily dose may be given as a single dose or in divided doses. The emulsion composition of the present invention can be administered systemically or locally via a transdermal route, a subcutaneous route, an intracutaneous route, an intramuscular route, a perineural route, an intrapulpal route, an intraspinal route, an epidural route, an intravenous route, or a transmucosal route such as eye mucosa etc. The dose can be changed by the skilled person in the art to obtain and/or maximize the therapeutic effect of the local anesthetic. In addition, to achieve the desired pain control, various anesthesia techniques, such as conduction anesthesia, infiltration anesthesia and topical anesthesia, can be employed using an appropriate dose. Conduction anesthesia is typified by an anesthesia technique in which an anesthetic is directly injected into, for example, peripheral nerves or the vicinity thereof to block stimulus transmission in the nervous system. Infiltration anesthesia is typified by an anesthesia technique in which an anesthetic is directly injected into, for example, a target tissue (into the skin, subcutis or the like surrounding the surgery site). Topical anesthesia is typified by an anesthesia technique used for blocking stimulus transmission in the nervous system in the superficial tissue, such as the skin or mucosa. Topical anesthesia is performed by topical application, spraying, surface freezing, or the like.

The blood level of a water-soluble medicinal ingredient can be measured by, for example, the method described in Japanese Patent No. 5723567, other known methods, or methods known per se.

The anesthetic effect of the local anesthetic in the emulsion composition of the present invention can be examined by applying stimulus to a human or animal subject and assessing whether the subject senses the stimulus. Specific examples of the method for examining anesthetic effect include the pinprick test.

The emulsion composition of the present invention is particularly excellent as a local anesthetic.

The emulsion composition of the present invention can have fast-acting property, but does not have sustained-release property.

More specifically, unlike sustained-release compositions, which are characterized in that gradual release of a medicinal substance after administration results in slow onset and long duration of action of and long-term maintenance of the blood level of the medicinal substance, the emulsion composition of the present invention is as capable of exerting immediate action as are commercially available aqueous solution compositions, and the elimination profile of the emulsion composition of the present invention in the blood is similar, or even superior in view of safety, to those of commercially available aqueous solution compositions.

The emulsion composition of the present invention can have a prolonged duration of action.

The emulsion composition of the present invention, as is clear from the Examples given below, (1) exerts immediate and long-lasting medicinal effect without sustained release after administration, and/or (2) has a high level of storage stability and safety.

The present invention includes embodiments in which the above-described features are combined differently within the technical scope of the present invention as long as these embodiments produce the effects of the present invention.

### EXAMPLES

Hereinafter, the present invention will be described in more detail by examples, but the present invention is not limited thereto. Many modifications can be made by persons of ordinary knowledge in the art within the scope of the technical idea of the present invention.

### (1) Emulsion composition production

### Production example

According to the ingredient compositions (mass ratio) shown in Tables 1 and 2 below, each sample was produced. More specifically, in the case of producing a 300-g sample, all the ingredients were weighed out in a 500-mL beaker, heated to 50°C, and subjected to crude emulsification with a homogenizing mixer (CLEARMIX (Japanese registered trademark), M Technique Co., Ltd.) at 12,000 rpm for 10 minutes. The crude emulsion was further subjected to processing with a high-pressure homogenizer (Beryu, BERYU) at a pressure of 0.5 to 0.7 Mpa 5 times or more to give an emulsion. In Comparative Examples 1 and 2, levobupivacaine hydrochloride was dissolved in physiological saline and used.

**[Table 1]**

| (g) | Comparative Example 1 | Comparative Example 2 | Example 1 | Example 2 | Example 3 |
|---|---|---|---|---|---|
| Levobupivacaine hydrochloride (in terms of levobupivacaine) | 0.282 (0.25) | 0.563 (0.5) | 0.282 (0.25) | 0.563 (0.5) | 0.563 (0.5) |
| Purified yolk lecithin | - | - | 1.2 | 1.2 | 1.2 |
| Medium-chain fatty acid glyceride (COCONARD MT) | - | - | 5 | 5 | 10 |
| Long-chain fatty acid glyceride (purified soybean oil) | - | - | 5 | 5 | - |
| Conc. glycerin | - | - | 2.25 | 2.25 | 2.25 |
| Water for injection | - | - | 86.268 | 85.987 | 85.987 |
| Physiological saline | 99.718 | 99.437 | - | - | - |
| pH | - | - | 5.24 | 5.07 | 4.94 |

**[Table 2]**

| (g) | Comparative Example 3 | Comparative Example 4 | Example 4 | Example 5 |
|---|---|---|---|---|
| Levobupivacaine hydrochloride (in terms of levobupivacaine) | - | - | 0.563 (0.5) | 0.563 (0.5) |
| Levobupivacaine (free) | 0.5 | 0.25 | - | - |
| Purified yolk lecithin | - | 1.2 | 1.2 | 1.2 |
| Medium-chain fatty acid glyceride (COCONARD MT) | 99.5 | 5 | 5 | 5 |
| Long-chain fatty acid glyceride (purified soybean oil) | - | 5 | 5 | 5 |
| Conc. glycerin | - | 2.25 | 2.25 | 2.25 |
| EDTA-2Na dihydrate (CLEWAT N) | - | - | 0.03 | - |
| EDTA-Ca-2Na dihydrate | - | - | - | 0.03 |
| Water for injection | - | 86.300 | 85.957 | 85.957 |
| pH | - | 7.66 | 5.02 | 5.19 |

### Test Example 1: Presence and absence of MCT and sensory nerve block duration

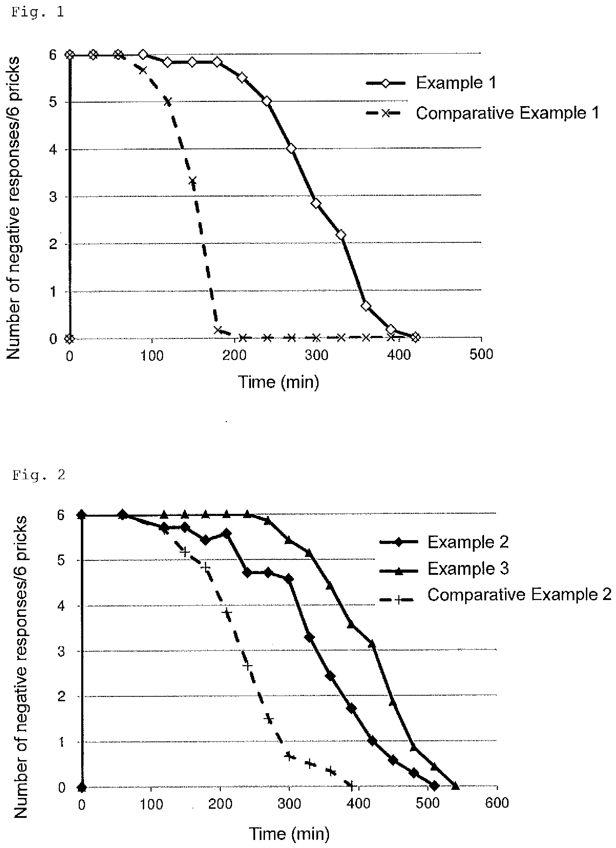

0.1 mL of the sample of Example 1 or Comparative Example 1 was intracutaneously administered from the shaved back skin of guinea pigs (Hartley, male, 5-week old, Japan SLC, Inc.). The guinea pigs were subjected to the pinprick test, specifically, the measurement of the number of responses to prinking with a syringe needle (6 pricks) to examine the sensory nerve block duration as a measure of local anesthetic effect (n = 6 per group). The results of the assessment of local anesthetic effect over 420 minutes after the intracutaneous administration of each sample are shown in Fig. 1.

The results of Fig. 1 show that the sample of Example 1, which was a lipid emulsion containing MCT, had a prolonged local anesthetic effect as compared with that of the sample of Comparative Example 1, which contained physiological saline as the solvent.

### Test Example 2: MCT content and sensory nerve block duration

The sensory nerve block duration was examined as described in Test Example 1 except for using the samples of Examples 2 and 3 and Comparative Example 2 (n = 6 per group) . The results of the assessment of local anesthetic effect over 540 minutes after the intracutaneous administration of each sample are shown in Fig. 2.

The results for Examples 2 and 3 and Comparative Example 2 in Fig. 2 show that the duration of the local anesthetic effect of levobupivacaine hydrochloride was prolonged with the increase of the amount of MCT added.

### Test Example 3: Oil-water partition ratio

5 g of an oil phase (soybean oil:medium-chain fatty acid triglyceride = 1:1) was added to 5 g of a 0.05% by mass aqueous levobupivacaine hydrochloride solution, and the mixture was stirred with a vortex mixer for 1 minute. After that, the whole was transferred to a centrifugation tube and centrifuged at 3600 rpm at 25°C for 10 minutes, and the aqueous phase was separated. The levobupivacaine concentration of the aqueous phase was measured by HPLC, and the oil-water partition ratio was determined. The results show that 94.3% of levobupivacaine hydrochloride was present in the aqueous phase.

### Test Example 4: Evaluation of sustained-release property

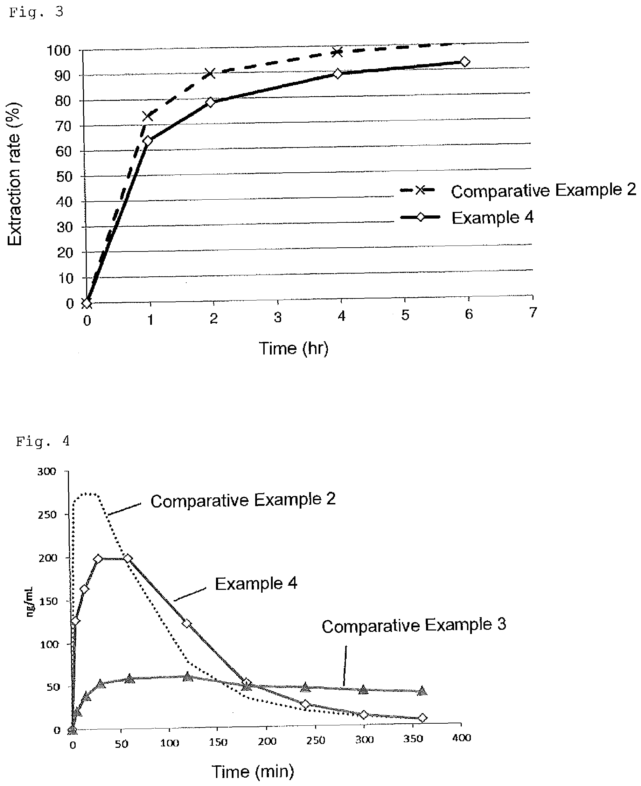

An aqueous glycerin solution and the sample of Example 4 or Comparative Example 2 were placed into the buffer chamber and the dialysis-membrane chamber of Single-Use RED Plate with Inserts (Thermo Fisher Scientific, Inc.), respectively, and the unit was covered with an adhesive film. After that, the unit was incubated on an orbital shaker at 100 rpm at 25°C. Aliquots of the aqueous glycerin solution were sampled over time, and the levobupivacaine concentrations in the aliquots were measured by HPLC to evaluate sustained-release property. The results of the time-course measurement of the extraction rate (%) of levobupivacaine in the aqueous glycerin solution are shown in Fig. 3.

The results for Example 4 and Comparative Example 2 in Fig. 3 show that MCT does not influence the sustained-release property of the composition of the present invention. To summarize the above, it was surprisingly found that, unlike the prior art, the composition of the present invention allows quick release of the active ingredient in spite of containing MCT and also has a prolonged duration of action as shown in the results of Test Examples 1 and 2.

Both the samples of Examples 2 and 4 were comparable in terms of fast-acting property, and had an equally prolonged duration of action as compared with that of the sample of Comparative Example 2 (data not shown). This indicates that EDTA-2Na dihydrate hardly influences the duration of action and sustained-release property.

### Test Example 5: Relation between emulsification and

### sustained-release property

0.1 mL of the sample of Example 4 or Comparative Example 2 or 3 was administered to the vicinity of the right sciatic nerve in rats (Sprague Dawley (SD), male, 7-week old, Japan SLC, Inc.), and blood samples were drawn from the jugular vein over time (n = 3 or 4 per group). The blood levobupivacaine level was measured according to the method described in Japanese

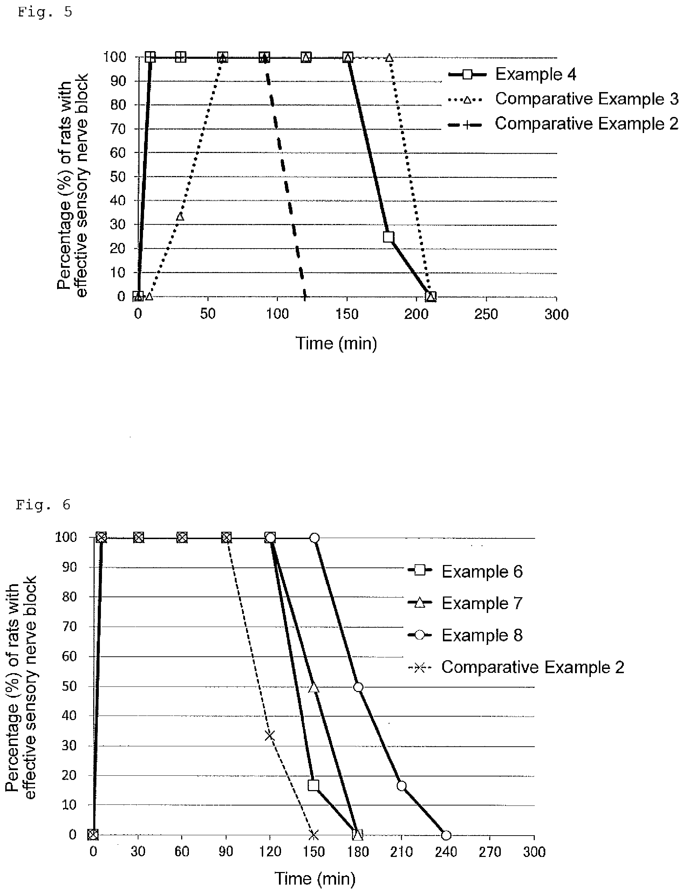

Patent No. 5723567. In addition, to evaluate anesthetic effect, measurement of the sensory nerve block duration was performed, more specifically, the middle fingers of both hindlimbs of each rat were pinched with forceps at given time points, and the presence or absence of withdrawal responses to the pinching was examined at each time point. The change in blood levobupivacaine level over 360 minutes after sample administration is shown in Fig. 4, and the results of the assessment of the duration of sensory nerve block effect are shown in Fig. 5.

Generally, an excessive increase of the blood levobupivacaine level has the risk of side effects (e.g., circulatory depression, convulsions, etc.). As is clear from Fig. 4, while the water-soluble composition (Comparative Example 2) showed a drastic elevation of the blood levobupivacaine level, the emulsion composition of the present invention (Example 4) showed a lower Cmax (maximum blood concentration) than that of the water-soluble composition (Comparative Example 2), which indicates that the emulsion composition of the present invention is safer. Both the compositions had a similar elimination profile of levobupivacaine in the blood.

In contrast, the lipophilic composition (Comparative Example 3) showed a slow elevation of the blood levobupivacaine level as compared with the emulsion composition (Example 4) and the water-soluble composition (Comparative Example 2), which demonstrates that the lipophilic composition has sustained-release property.

As is clear from Fig. 5, while the lipophilic composition (Comparative Example 3) showed a slow onset of action, the emulsion composition of the present invention (Example 4) showed a fast onset of action without delay upon administration, as with the water-soluble composition (Comparative Example 2) . Also found in Fig. 5 was that the emulsion composition of the present invention (Example 4) showed a prolonged duration of action as compared with the water-soluble composition (Comparative Example 2).

In addition, although the lipophilic composition (Comparative Example 3) no longer showed sensory nerve block effect 200 minutes after administration or later (Fig. 5), the blood levobupivacaine level was higher than those of the other samples (Fig. 4). That is, levobupivacaine in the lipophilic composition remained in the blood at a relatively high level without exerting medicinal action. In contrast, the emulsion composition of the present invention (Example 4) showed a reduction in blood levobupivacaine level from after the sensory nerve block effect disappeared as with the water-soluble composition (Comparative Example 2), which demonstrates that the blood levobupivacaine level changed in a safer manner.

To summarize the above, it was surprisingly found that the composition of the present invention enables fast as well as prolonged local anesthetic action of the active ingredient and allows the blood level of the active ingredient to change in a safer manner without drastic elevation as compared with the aqueous solution composition (Comparative Example 2).

### Test Example 6: Storage stability

The storage stability of the samples of Example 1 and Comparative Example 4 was examined at 25°C/60%RH. As shown in Table 3, the residual rate of levobupivacaine in the sample of Example 1 was maintained at 98% or more even after 6-month storage, but the residual rate of levobupivacaine in the sample of Comparative Example 4, which had an initial pH of about 7.7, decreased over time, and after 6 months, was as low as about 85%.

**[Table 3]**

| Levobupivacaine content (residual rate: %) | | | | |
|---|---|---|---|---|
| | 0M | 2M | 4M | 6M |
| Example 1 | 100.0 | 101.3 | 101.8 | 98.4 |
| Comparative Example 4 | 100.0 | 97.5 | 92.0 | 85.1 |

### Test Example 7: Emulsification stability

Based on the formulation of Example 2, various samples were produced with alterations, specifically, addition of 0.0055% by mass, 0.016% by mass, or 0.03% by mass EDTA-2Na dihydrate, or addition of 0.03% by mass EDTA-Ca-2Na, and reduction of the corresponding amount of distilled water. These samples were sterilized (121°C, 20 minutes) and then stored at a constant temperature for 6 months, during which the appearance of each sample was visually observed at regular intervals for stability evaluation.

### (1) Results for sample with basic formulation

There was no change in visual appearance at 25°C for 3 months, and long-term storage stability was demonstrated.

### (2) Results for sample containing 0.0055% by mass or 0.016% by mass EDTA-2Na dihydrate

There was no change in visual appearance at 25°C for 4 months, and longer-term storage stability was obtained by the addition of EDTA.

### (3) Results for sample containing 0.03% by mass EDTA-2Na dihydrate (Example 4) or 0.03% by mass EDTA-Ca-2Na dihydrate

### (Example 5)

There was no change in visual appearance at 5°C, 25°C or 40°C for 6 months, and further longer-term storage stability was obtained by the addition of higher-concentration EDTA. Moreover, the addition of EDTA prevented change in color tone of the visual appearance (see Table 4).

**[Table 4]**

| Emulsification stability (visual appearance at 25°C) | | | |
|---|---|---|---|
| | 0M | 3M | 6M |
| Example 2 | White emulsion | Slightly reddish white emulsion | Slightly reddish white emulsion |
| Example 3 | White emulsion | White emulsion | White emulsion |
| Example 4 | White emulsion | White emulsion | White emulsion |

### (2) Emulsion composition production 2

### Production example 2

According to the ingredient compositions (mass ratio) shown in Table 5 below, each sample was produced. More specifically, in the case of producing a 300-g sample, all the ingredients were weighed out in a 500-mL beaker, heated to 50°C, and subjected to crude emulsification with a homogenizing mixer (CLEARMIX (Japanese registered trademark), M Technique Co., Ltd.) at 12,000 rpm for 10 minutes. The crude emulsion was further subjected to processing with a high-pressure homogenizer (Beryu, BERYU) at a pressure of 0.5 to 0.7 Mpa 5 times or more to give an emulsion. In addition, the sample of Comparative Example 2 shown in Table 1 was produced and used as a reference.

**[Table 5]**

| | Example 6 | Example 7 | Example 8 |
|---|---|---|---|
| Levobupivacaine hydrochloride (in terms of levobupivacaine) | 0.563 (0.5) | 0.563 (0.5) | 0.563 (0.5) |
| Purified yolk lecithin | 1.2 | 1.2 | 1.2 |
| Medium-chain fatty acid glyceride (COCONARD RK; caprylic triglyceride) | 5 | - | - |
| Medium-chain fatty acid glyceride (Sunfat GDC-S; caprylic diglyceride) | - | 5 | - |
| Medium-chain fatty acid glyceride (Captex 100; propylene glycol dicaprate) | - | - | 5 |
| Long-chain fatty acid glyceride (purified soybean oil) | 5 | 5 | 5 |
| Conc. Glycerin | 2.25 | 2.25 | 2.25 |
| EDTA-2Na dihydrate (CLEWAT N) | 0.03 | 0.03 | 0.03 |
| Distilled water (Otsuka distilled water) | 85.957 | 85.957 | 85.957 |
| pH | 4.97 | 5.12 | 4.96 |

### Test Example 8: Effect of various fatty acid esters

0.1 mL of the sample of any of Examples 6 to 8 or the sample of Comparative Example 2 was administered to the vicinity of the right sciatic nerve in rats (Sprague Dawley (SD), male, 7-week old, Japan SLC, Inc.) (n = 6 per group). To evaluate anesthetic effect, measurement of the sensory nerve block duration was performed, more specifically, the middle fingers of both hindlimbs of each rat were pinched with forceps at given time points, and the presence or absence of withdrawal responses to the pinching was examined at each time point. The results of the assessment of the duration of sensory nerve block effect after sample administration are shown in Table 6 and Fig. 6.

As is clear from Fig. 6, the emulsion compositions of the present invention (Examples 6 to 8) showed a fast onset of action as with the water-soluble composition (Comparative Example 2) . Also found in Fig. 6 was that the emulsion compositions of the present invention (Examples 6 to 8) showed a prolonged duration of action as compared with the water-soluble composition (Comparative Example 2).

To summarize the above, it was surprisingly found that the compositions of the present invention enable fast as well as prolonged local anesthetic action of the active ingredient.

Although data are not shown, the pH, residual rate (%), particle size (nm) and visual appearance of the samples of the above Examples during the storage at 25°C or 40°C/75%RH for 2 months or longer were also constantly maintained.

**[Table 6]**

| | Mean of anesthesia duration (min) |
|---|---|
| Comparative Example 2 | 100 |
| Example 6 | 125 |
| Example 7 | 135 |
| Example 8 | 170 |

### INDUSTRIAL APPLICABILITY

The emulsion composition of the present invention is useful as a pharmaceutical preparation comprising a local anesthetic as an active ingredient.

## Claims

1. An acidic emulsion composition comprising a local anesthetic and a fatty acid ester containing an ester-bonded fatty acid having 6 to 12 carbon atoms.

2. The acidic emulsion composition according to claim 1, wherein the fatty acid ester is a glyceride.

3. The acidic emulsion composition according to claim 1 or 2, wherein the local anesthetic is an amide-type local anesthetic.

4. The acidic emulsion composition according to any one of claims 1 to 3, wherein the local anesthetic is one or more selected from the group consisting of levobupivacaine, bupivacaine, ropivacaine and salts thereof.

5. The acidic emulsion composition according to any one of claims 2 to 4, wherein the glyceride is one or more fatty acid glycerides comprising one or more fatty acids having 6 to 12 carbon atoms as a constituent fatty acid, and is selected from the group consisting of a fatty acid monoglyceride, a fatty acid diglyceride and a fatty acid triglyceride.

6. The acidic emulsion composition according to any one of claims 1 to 5, wherein the emulsion composition has a pH of 3 or higher but lower than 7.0.

7. The acidic emulsion composition according to any one of claims 1 to 5, wherein the emulsion composition has a pH of 3 to 6.5.

8. The acidic emulsion composition according to any one of claims 1 to 5, wherein the emulsion composition has a pH of 3 to 6.0.

9. The acidic emulsion composition according to any one of claims 1 to 8, further comprising EDTA.

10. The acidic emulsion composition according to any one of claims 1 to 9, further comprising a long-chain fatty acid glyceride.

11. The acidic emulsion composition according to any one of claims 1 to 10, wherein the emulsion is an O/W type emulsion.

12. The acidic emulsion composition according to claim 11, wherein the proportion of the local anesthetic in an aqueous phase is 15% or more of the total amount of the local anesthetic in the composition.

13. The acidic emulsion composition according to any one of claims 1, 3, 4, and 6 to 12, wherein the fatty acid ester is a propylene glycol fatty acid ester.

14. The acidic emulsion composition according to any one of claims 1 to 13 for use in pain control in a mammal.

15. The composition for use according to claim 14, wherein the pain control is achieved by systemic or local administration via a transdermal route, a subcutaneous route, an intracutaneous route, an intramuscular route, a perineural route, an intrapulpal route, an intraspinal route, an epidural route, an intravenous route, or a transmucosal route such as eye mucosa etc.

16. The composition for use according to claim 14, wherein the pain control is achieved by conduction anesthesia, infiltration anesthesia or topical anesthesia.
